# EUROPÄISCHE PATENTSCHRIFT

(11) **EP 2 866 684 B1**
(45) Veröffentlichungstag und Bekanntmachung des Hinweises auf die Patenterteilung: **06.09.2017**
(21) Anmeldenummer: 13728984.9
(22) Anmeldetag: 28.05.2013
(51) Int. Cl.: A61B 17/122

(54) **CHIRURGISCHER CLIP MIT INNENFEDER**
SURGICAL CLIP WITH INNER SPRING
PINCE CHIRURGICALE À RESSORT INTERNE

(30) Priorität: 29.06.2012 DE 102012211379
(43) Veröffentlichungstag der Anmeldung: 06.05.2015
(73) Patentinhaber: Aesculap AG, 78532 Tuttlingen (DE); Karlsruher Institut für Technologie, 76131 Karlsruhe (DE)
(72) Erfinder: PLEIL, Thomas, 78073 Bad Dürrheim (DE); TESARI, Iwiza, 76344 Eggenstein-Leopoldshafen (DE); STEINHILPER, Klaus-Dieter, 78532 Tuttlingen (DE); NESPER, Markus, 78532 Tuttlingen (DE)
(74) Vertreter: Koller, Tobias Kilian
(86) Internationale Anmeldenummer: PCT/EP2013/060924
(87) Internationale Veröffentlichungsnummer: WO 2014/001008

(56) Entgegenhaltungen:
- EP-A1- 0 105 414
- WO-A2-01/35832
- US-A1- 2010 036 398
- US-B1- 6 179 850

## Beschreibung

Die vorliegende Erfindung betrifft einen Clip mit Innenfeder.

### Stand der Technik

Im Stand der Technik gibt es mehrere Ansätze, um die Schließkraft eines chirurgischen Clips zu steuern bzw. zu erhöhen. Eine einfache Möglichkeit, die Schließkraft eines chirurgischen Clips zu erhöhen, besteht darin, bei einem Clip mit Schenkelfeder die Anzahl der Wicklungen der Schenkelfeder zu erhöhen. Es kann aber auch der Querschnitt der Schenkelfeder verändert werden, sodass das Flächenträgheitsmoment der Feder zunimmt. Das Flächenträgheitsmoment der Schenkelfeder, welche eine auf Biegung beanspruchte Feder ist, bestimmt zusammen mit dem E-Modul der Schenkelfeder den Verlauf der elastischen Verformung bzw. Auslenkung in Abhängigkeit von der aufgebrachten Kraft bzw. dem aufgebrachten Moment. Die Druckschrift US6179850 offenbart einen chirurgischen Clip mit den Merkmalen des Oberbegriffs von Anspruch 1. Durch die Tatsache, dass im medizinischen Bereich die verwendeten Materialien ausgiebig getestet und zugelassen sein müssen, stehen nur relativ wenige Materialien zur Verfügung. Eine Veränderung des Querschnitts der Schenkelfeder lohnt sich vor allem dann, wenn die Höhe des gewickelten Querschnitts geändert wird, da die Höhe einen weit größeren Einfluss auf das Flächenträgheitsmoment hat als die Breite dieses Querschnitts. Eine Veränderung dieses Querschnitts bringt aber auch Nachteile mit sich. Zum einen verkleinert sich dadurch der Bereich, in dem die Feder verformt werden kann, ohne dass ein Plastizieren derselben eintritt, und zum anderen vergrößern sich dadurch die Abmessungen der Feder. Auch bei dem Einsatz von mehr Wicklungen vergrößern sich die Abmessungen der Feder.

Eine andere Möglichkeit, die Federkraft eines chirurgischen Clips zu erhöhen, liegt darin, einen Verstärkungsclip auf den chirurgischen Clip zu applizieren. Dazu werden zumeist Clips mit seitlich gebogenen Branchen verwendet, die auf die Branchen des chirurgischen Clips aufgesetzt werden und somit den chirurgischen Clip verstärken. Diese Vorgehensweise benötigt aber mehr Platz als ein einzelner Clip, birgt die Gefahr, dass der Verstärkungsclip sich von dem chirurgischen Clip löst, und verteuert den Vorgang erheblich, zum einen dadurch, dass zwei Clips verwendet werden müssen und zum anderen dadurch, dass beide Clips nacheinander appliziert werden müssen.

Die Aufgabe der vorliegenden Erfindung ist es daher, einen chirurgischen Clip bereit zu stellen, der eine erhöhte Federkraft aufweist, ohne dass seine äußeren Abmessungen zunehmen.

Die Aufgabe der vorliegenden Erfindung wird gelöst durch einen chirurgischen Clip gemäß Anspruch 1. Weitere vorteilhafte Ausgestaltungen sind Gegenstand der abhängigen Ansprüche.

Gemäß einem Aspekt der vorliegenden Erfindung weist ein chirurgischer Clip zwei Clipbranchen, die jeweils einen Klemmabschnitt und einen Betätigungsabschnitt aufweisen, sowie eine Biegefederanordnung auf, über die die beiden Clipbranchen einstückig miteinander gekoppelt sind. Die beiden Clipbranchen und die Biegefederanordnung können dabei aus einem Material und einem Stück gefertigt sein, sie können aber auch aus verschiedenen Materialien hergestellt sein, die anschließend zusammengeführt werden. Darüber hinaus weist die Biegefederanordnung mehr als eine halbe Wicklung auf und ein elastischer Körper ist so in der Biegefederanordnung angeordnet, dass er durch einen Öffnungsvorgang des chirurgischen Clips verformbar ist. Zwischen dem elastischen Körper und der Biegefederanordnung sind drei oder mehr Kontaktstellen ausgebildet, wobei die drei oder mehr Kontaktstellen über einen Winkelbereich von mindestens 180° verteilt sind

Wenn die Biegefederanordnung nicht mehr als eine halbe Wicklung aufweist, ist es nicht möglich, einen elastischen Körper in der Biegefederanordnung so anzuordnen, dass er dort sicher verbleibt, da er sonst zumindest seitlich aus der Biegefederanordnung austreten bzw. entweichen kann. Vorzugsweise weist der Clip etwa eineinhalb Wicklungen auf. Der elastische Körper ist dann erfindungsgemäß in der Biegefederanordnung angeordnet, wenn der elastische Körper verformt wird, sobald der chirurgische Clip geöffnet wird. Wenn der chirurgische Clip geöffnet wird, also die Klemmabschnitte voneinander weg bewegt werden, verkleinert sich der Innendurchmesser der Biegefederanordnung. Diese Verkleinerung des Innendurchmessers der Biegefederanordnung wirkt auf den elastischen Körper ein, was zu einer Verformung des elastischen Körpers und zu einer Erhöhung der Federkraft des chirurgischen Clips führt.

Gemäß einer vorteilhaften Ausgestaltung dieses Aspekts der Erfindung ist der elastische Körper ein im Wesentlichen C-förmiger elastischer Körper. Dieser C-förmige Körper ist so in der Biegefederanordnung angeordnet, dass er sich im Wesentlichen entlang seiner gesamten Außenfläche in Kontakt mit der Innenfläche der Biegefederanordnung befindet. Im Wesentlichen entlang seiner gesamten Außenfläche bedeutet hier nicht, dass die gesamte oder annähernd gesamte Außenfläche in Kontakt mit der Innenfläche der Biegefederanordnung sein muss. Bei einer Biegefederanordnung, die aus einem runden Profil hergestellt ist, bilden sich zwischen der Biegefederanordnung und dem C-förmigen Körper Kontaktlinien aus. Im Prinzip bildet sich eine spiralförmige Kontaktlinie, welche dort unterbrochen ist, wo der Körper in axialer Richtung geschlitzt ist, um die C-Form zu erhalten. Im Wesentlichen entlang seiner gesamten Außenfläche bedeutet hier also, dass der elastische Körper im Wesentlichen entlang seines gesamten Umfangs Kontaktstellen mit der Biegefederanordnung ausbildet.

Alternativ dazu ist es auch möglich, dass die Außenfläche des elastischen Körpers nur an einigen wenigen Stellen in Kontakt mit der Innenfläche der Biegefederanordnung gelangt. Auch in diesem Fall würde der elastische C-förmige Körper als Federkraftverstärkung für den chirurgischen Clip dienen. Allerdings kann es bei solch einem Aufbau sein, dass versehentlich Gewebe zwischen der Biegefederanordnung und dem elastischen Körper eingeklemmt wird, da sich in diesem Fall der Abstand zwischen der Außenfläche des elastischen Körpers und der Innenfläche der Biegefederanordnung verändert.

Die erfindungsgemäß ausgebildeten drei oder mehr Anlage- bzw. Kontaktstellen können frei entlang der Innenfläche der Biegefederanordnung verteilt sein, vorausgesetzt, dass diese sich über einen Winkelbereich von mindestens 180° erstrecken. D.h. verbindet man den ersten Kontaktpunkt des elastischen Körpers mit der Biegefederanordnung mit dem Mittelpunkt der Biegefederanordnung und den letzten Kontaktpunkt des elastischen Körpers mit der Biegefederanordnung ebenfalls mit dem Mittelpunkt der Biegefederanordnung, so müssen diese beiden Verbindungslinien einen Winkel von mindestens 180° einschließen. Andernfalls gäbe es keine Klemmwirkung zwischen dem elastischen Körper und der Biegefederanordnung und der elastische Körper würde nicht als Federverstärkung dienen und würde aus dem Innenbereich der Biegefederanordnung herausfallen.

Gemäß einer anderen vorteilhaften Ausgestaltung dieses Aspekts der Erfindung ist der im Wesentlichen C-förmige elastische Körper zu den Klemmabschnitten der Clipbranchen hin offen. Dies bedeutet, dass der Schlitz in der Mantelfläche, welcher den C-förmigen Körper von einem hohlzylindrischen Körper unterscheidet, zu der Mitte des Mauls des chirurgischen Clips zweigt.

Alternativ kann der Schlitz auch von der Mitte des Mauls weg zeigen oder in einer beliebigen Richtung dazu angeordnet sein. Die Ausrichtung des Schlitzes zur Maulmitte hin ermöglicht eine bessere Sicht in Richtung des Mauls und sorgt zudem für eine gleichmäßige Federkraftverstärkung.

Gemäß einer weiteren vorteilhaften Ausgestaltung dieses Aspekts der Erfindung ist der elastische Körper ein im Wesentlichen stabförmiger elastischer Körper ist, der so in der Biegefederanordnung angeordnet ist, dass die Kontaktstellen zwischen der Mantelfläche des elastischen stabförmigen Körpers und der Innenfläche der Biegefederanordnung entlang des gesamten Umfangs des elastischen stabförmigen Körpers vorzugsweise gleichmäßig verteilt sind. Wie bereits oben für den C-förmigen elastischen Körper erläutert bedeutet dies nicht, dass die gesamte Mantelfläche des elastischen Körpers in Kontakt mit der Biegefederanordnung stehen muss, sondern die Kontaktstellen zwischen Mantelfläche des elastischen Körpers und der Innenfläche der Biegefederanordnung befinden sich im Wesentlichen entlang des gesamten Umfangs des elastischen Körpers. Insbesondere muss sich eine Kontaktstelle nicht über die gesamte axiale Länge der Außenfläche oder Mantelfläche des elastischen Körpers erstrecken.

Gemäß einer vorteilhaften Ausgestaltung dieses Aspekts der Erfindung weist der im Wesentlichen stabförmige elastische Körper einen kreisförmigen, einen vieleckigen oder einen sternförmigen Querschnitt auf. Dabei kann es sich um einen regelmäßigen oder unregelmäßigen vieleckigen oder sternförmigen Querschnitt handeln. Zudem kann der stabförmige elastische Körper nicht nur die Querschnittsform eines Sternpolygons aufweisen sondern auch die eines Sterns, der durch Umkreis, Inkreis und Strahlenanzahl definiert ist (Beispiele: Natostern, Mercedesstern). Im Falle eines kreisförmigen Querschnitts bildet sich eine umlaufende Kontaktstelle zwischen der Außenfläche bzw. Mantelfläche des elastischen Körpers und der Innenfläche der Biegefederanordnung aus. Bei einem vieleckigen oder sternförmigen Querschnitt bildet sich eine entsprechende Anzahl einzelner Kontaktstellen aus, die bei regelmäßigen Querschnitten gleichmäßig entlang des Umfangs des elastischen Körpers verteilt sind, andernfalls ungleichmäßig.

Gemäß noch einer vorteilhaften Ausgestaltung dieses Aspekts der Erfindung ist der elastische Körper ein Federkäfig, der so in der Biegefederanordnung angeordnet ist, dass er sich im Wesentlichen entlang seines gesamten Umfangs mit seiner Außenfläche in Kontakt mit der Innenfläche der Biegefederanordnung befindet. Bei dieser Ausbildung werden nur dort Kontaktstellen zwischen der Außenfläche des Federkäfigs und der Innenfläche der Biegefederanordnung ausgebildet, wo ein Gitterstab die Außenfläche des Federkäfigs definiert. Vorzugsweise sind die Gitterstäbe des Federkäfigs entlang seines Umfangs gleichmäßig verteilt.

Gemäß einer weiteren vorteilhaften Ausgestaltung dieses Aspekts der Erfindung ist an wenigstens einer Stirnfläche des elastischen Körpers eine Halteeinrichtung vorgesehen, welche über den Innenradius der Biegefederanordnung nach außen vorsteht. Mit einer solchen Halteeinrichtung kann sicher gestellt werden, dass der elastische Körper nicht unbeabsichtigt aus dem Innenbereich der Biegefederanordnung entweichen kann. Die Halteeinrichtung besteht beispielsweise aus zwei Vorsprüngen, die diametral gegenüber liegend an dem Außenrand der Stirnfläche des elastischen Köpers vorgesehen sind. Alternativ dazu können aber auch drei oder mehr Vorsprünge vorgesehen sein, die sich in einem Winkelbereich von mindestens 180° entlang des Außenrands der Stirnfläche befinden.

Bevorzugt ist an beiden Stirnflächen des elastischen Körpers eine Halteeinrichtung vorgesehen. Eine Halteeinrichtung kann beispielsweise einstückig mit dem elastischen Körper ausgebildet werden oder an dem elastischen Körper befestigt werden, bevor dieser in der Biegefederanordnung angeordnet wird. Die andere Halteeinrichtung oder aber beide Halteeinrichtungen wird/werden dann per Kleben, Schrauben, Schweißen oder anderweitig an dem elastischen Körper angebracht, nachdem der elastische Körper in der Biegefederanordnung angeordnet wurde. Vorzugsweise ist der Abstand der Halteeinrichtung zu den Seitenflächen der Biegefederanordnung gering. Ein gewisser Spalt zwischen der Halteeinrichtung und den Seitenflächen der Biegefederanordnung sollte aber gewahrt bleiben, um keine unerwünschte Reibung zwischen diesen Bauteilen zu erzeugen.

Gemäß einer vorteilhaften Ausgestaltung dieses Aspekts der Erfindung erstreckt sich die Halteeinrichtung im Wesentlichen entlang des gesamten Außenumfangs des elastischen Körpers. Die Halteeinrichtung ist quasi ein ringförmiges Element, welches eine Art Bund bildet. Der Bund bzw. die Halteeinrichtung muss sich aber nicht entlang des gesamten Umfangs der Biegefederanordnung erstrecken. Bei einem C-förmigen elastischen Körper erstreckt sich die Halteeinrichtung vorzugsweise entlang der gesamten Stirnfläche des C-förmigen Körpers. Die Halteeinrichtung kann aber auch nur an bestimmten Abschnitten der Stirnfläche des elastischen Körpers befinden, beispielsweise im Bereich beider Enden und des mittleren Abschnitts des C-förmigen elastischen Körpers.

Gemäß einer weiteren, besonders vorteilhaften Ausgestaltung dieses Aspekts der Erfindung weist der elastische Körper einen in axialer Richtung veränderlichen Querschnitt derart auf, dass er beiderseits der Biegefederanordnung einen gegenüber dem zentralen Bereich, an dem er sich in Kontakt mit der Innenfläche der Biegefederanordnung befindet, vergrößerten Umkreis aufweist. Dies bedeutet, dass der elastische Körper in dem Bereich, in dem sich die Biegefederanordnung befindet, eine im Wesentlichen umlaufende Nut ausbildet. Die Biegefederanordnung befindet sich zumindest teilweise in dieser Nut und die Innenfläche der Biegefederanordnung befindet sich zumindest teilweise in Kontakt mit der Bodenfläche der umlaufenden Nut. Im Falle eines C-förmigen elastischen Körpers kann dieser ebenfalls mit einer Nut versehen sein. Zum Einbringen dieses C-förmigen Körpers in die Biegefederanordnung muss der C-förmige Körper nur etwas weiter zusammen gebogen werden, als dies in der Grundstellung des chirurgischen Clips der Fall ist. Anschließend wird er seitlich in die Biegefederanordnung eingeschoben und entspannt, wodurch er sich an die Innenfläche der Biegefederanordnung anlegt. Bei korrekter Positionierung des C-förmigen elastischen Körpers rastet die Biegefederanordnung in die umlaufende Nut des C-förmigen Körpers ein und sichert so dessen Lage in der Biegefederanordnung.

Gemäß einer besonderen Ausbildung dieses elastischen Körpers mit axial veränderlichem Querschnitt kann eine Nut derart ausgebildet sein, dass die eine kerbenartige Vertiefung in der Außenumfangsfläche des elastischen Körpers ausbildet. es wird in diesem Fall also eine V-förmige umlaufende Nut geschaffen. In diesem Fall wird ein selbstzentrierender C-förmiger elastischer Körper geschaffen, dessen umlaufende Nut zwei schräge Flächen aufweist. Da der C-förmige elastische Körper zu einem gewissen Grad vorgespannt ist, zumindest wenn die Biegefederanordnung an dessen Stirnbereich anliegt, gewährleisten die schrägen Flächen, dass sich der C-förmige elastische Körper gegenüber der Biegefederanordnung selbst zentriert, da er in dieser Position, also wenn die Biegefederanordnung im zentralen Bereich des C-förmigen elastischen Körpers anliegt, eine geringere Vorspannung aufweist als wenn die Biegefederanordnung in einem Stirnbereich des C-förmigen elastischen Körpers anliegt. Die Nut kann aber auch U-förmig sein oder es kann eine im Wesentlichen V-förmige Nut mit gekrümmten Seitenwänden geschaffen werden. Zwischen der Bodenfläche der umlaufenden Nut und der Mantelfläche des elastischen Körpers kann auch eine Stufe ausgebildet sein.

Gemäß einer besonders vorteilhaften Ausgestaltung dieses Aspekts der Erfindung weist die Biegefederanordnung eineinhalb Wicklungen auf. Eineinhalb Wicklungen sind besonders vorteilhaft, da sie einerseits sicher stellen, dass der elastische Körper entlang seines gesamten Umfangs in Kontakt mit der Innenfläche der Biegefederanordnung gelangen kann, und andererseits die Breite der Biegefederanordnung nicht übermäßig vergrößern.

Gemäß einer weiteren, besonders vorteilhaften Ausgestaltung dieses Aspekts der Erfindung sind die beiden Clipbranchen zwischen dem jeweiligen Klemmabschnitt und dem jeweiligen Betätigungsabschnitt verschränkt. Dies bedeutet, dass die Biegefederanordnung aus einer ungeraden Anzahl von halben Wicklungen besteht ([2k+1]/2, wobei k eine nichtnegative ganze Zahl ist) und das Maul des chirurgischen Clips geöffnet wird, indem die Wicklungszahl tendenziell erhöht wird, d.h. die Biegefederanordnung weiter eingewickelt wird. Dabei verringert sich der Innendurchmesser der Biegefederanordnung, wodurch eine ringförmige Druckkraft auf den elastischen Körper in der Biegefederanordnung aufgebracht wird.

Gemäß noch einer vorteilhaften Ausgestaltung dieses Aspekts der Erfindung ist eine der beiden Clipbranchen im Bereich der Verschränkung geschlitzt und die andere der beiden Clipbranchen erstreckt sich durch diesen Schlitz und ist durch den Schlitz geführt. Auf diese Weise kann verhindert werden, dass die Federlast exzentrisch in die Klemmabschnitte eingeleitet wird und dazu führt, dass sich die beiden Klemmabschnitte über die Grundstellung hinaus aneinander vorbei bewegen und somit ein sicheres Schließen des chirurgischen Clips nicht gewährleistet werden kann.

Gemäß einer vorteilhaften Ausgestaltung dieses Aspekts der Erfindung ist der Clip zumindest im Bereich der Biegefederanordnung beschichtet oder lackiert, um die Reibung zwischen der Biegefederanordnung und dem elastischen Körper zu verringern. Auf diese Weise kann aber auch die Reibung innerhalb der einzelnen Wicklungen der Biegefederanordnung verringert werden oder aber auch die Reibung zwischen der Biegefederanordnung und einem Applikationsinstrument, welches den chirurgischen Clip an der Biegefederanordnung greift und öffnet, indem es die Biegefederanordnung zusammen drückt.

Gemäß einer weiteren vorteilhaften Ausgestaltung dieses Aspekts der Erfindung ist der elastische Körper aus Kunststoff oder Metall ausgebildet. Vorzugsweise ist der elastische Körper aus Federstahl oder einer Titanlegierung ausgebildet, wenn es sich um einen C-förmigen elastischen Körper oder einen Gitterkörper handelt. Bei einem stabförmigen elastischen Körper kann auch ein festes Gummimaterial (beispielsweise Silikon) gewählt werden, unabhängig davon, ob der Querschnitt kreisförmig, vieleckig oder sternförmig gewählt ist. Selbstverständlich können auch Materialkombinationen verwendet werden, beispielsweise ein metallischer C-förmiger Körper mit einer ringförmigen Halteeinrichtung aus Kunststoff.

Weitere Vorteile und Merkmale der Erfindung sind dem Fachmann aus den beigefügten Figuren und der detaillierten Beschreibung der Ausführungsbeispiele ersichtlich.
- Fig. 1A: ist eine seitliche Abbildung eines ersten Ausführungsbeispiels der vorliegenden Erfindung;
- Fig. 1B: ist eine Darstellung eines C-förmigen elastischen Körpers entsprechend Fig. 1A;
- Fig. 1C: ist eine seitliche Abbildung eines zweiten Ausführungsbeispiels der vorliegenden Erfindung;
- Fig. 1D: ist eine Darstellung eines C-förmigen elastischen Körpers entsprechend Fig. 1C;
- Fig. 2: ist eine seitliche Abbildung eines dritten Ausführungsbeispiels der vorliegenden Erfindung;
- Fig. 3: ist eine seitliche Abbildung eines vierten Ausführungsbeispiels der vorliegenden Erfindung; und
- Fig. 4: ist eine Darstellung eines C-förmigen elastischen Körpers gemäß einem fünften Ausführungsbeispiel.

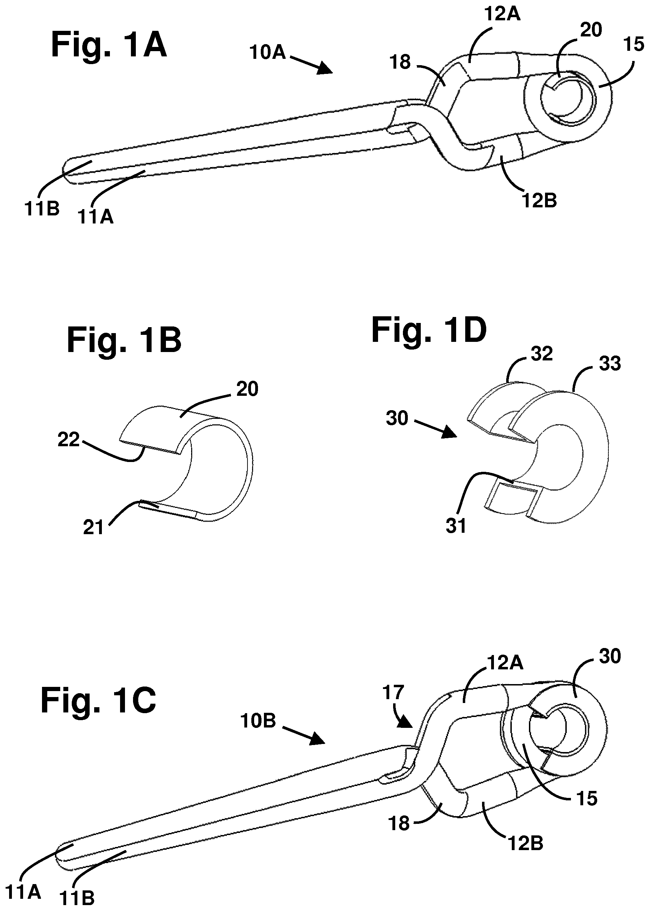

Ein erstes Ausführungsbeispiel der vorliegenden Erfindung ist im Folgenden unter Bezugnahme auf die Fig. 1A und 1B im Detail beschrieben.

Der chirurgische Clip 10A gemäß dem ersten Ausführungsbeispiel hat zwei Clipbranchen. Jede Clipbranche weist einen Klemmabschnitt 11A, 11B und einen Betätigungsabschnitt 12A, 12B auf. Der chirurgische Clip 10A hat zudem eine Biegefederanordnung 15, über welche die beiden Clipbranchen einstückig miteinander gekoppelt sind.

Die Biegefederanordnung 15 dieses Ausführungsbeispiels weist eine eineinhalbfache Wicklung auf und ein C-förmiger elastischer Körper 20, der in Fig. 1B im Detail gezeigt ist, ist in der Biegefederanordnung 15 angeordnet. Der C-förmige elastische Körper 20 liegt mit seiner gesamten Außenumfangsfläche 21 an der Innenumfangsfläche der Biegefederanordnung 15 an. Der C-förmige elastische Körper 20 ist so angeordnet, dass er zu den Klemmabschnitten 11A, 11b hin offen ist, d.h. der Schlitz in dem C-förmigen Körper 20 zeigt zur Maulmitte hin.

Der C-förmige elastische Körper 20 ist zudem entlang seines Umfangs gleichmäßig dick. In einer anderen Ausführungsform kann die Dicke des elastischen Körpers 21 auch unterschiedlich sein. Der elastische Körper 20 kann aus verschiedenen Materialien ausgebildet sein, beispielsweise Federstahl, Kunststoff oder einer Titanlegierung. Im vorliegenden Fall ist er wie der gesamte chirurgische Clip 10A aus einer Titanlegierung ausgebildet.

Bei dem chirurgischen Clip 10A gemäß diesem Ausführungsbeispiel sind die beiden Clipbranchen zwischen dem jeweiligen Klemmabschnitt 11A, 11B und dem jeweiligen Betätigungsabschnitt 12A, 12B verschränkt. Dabei schließt sich der Klemmabschnitt 11A an den Betätigungsabschnitt 12A an und der Klemmabschnitt 11B schließt sich an den Betätigungsabschnitt 12B an. Zwischen dem Betätigungsabschnitt 12A und dem Klemmabschnitt 11A ist ein Abschnitt 18 mit verringerter Breite ausgebildet. Zwischen dem Betätigungsabschnitt 12B und dem Klemmabschnitt 11B ist ein Schlitzabschnitt 17 ausgebildet. Der Abschnitt 18 mit verringerter Breite und der Schlitzabschnitt sind miteinander verschränkt, wobei sich der Abschnitt 18 mit verringerter Breite durch den Schlitzabschnitt 17 erstreckt. Auf diese Weise sind die beiden Klemmabschnitte 11A und 11B zueinander geführt. Darüber hinaus weist die Biegefederanordnung 15 eine Beschichtung auf. Im Bereich des Übergangs von der Biegefederanordnung 15 zu den beiden Betätigungsabschnitten 12A und 12B sind Verdickungen vorgesehen, die ein Greifen mit einem Applikatorinstrument vereinfachen sollen.

Der C-förmige elastische Körper 20 dieses Ausführungsbeispiels weist keine Nut oder Halteeinrichtung auf. Er wird in die Biegefederanordnung 15 eingebracht bzw. eingesetzt, indem er zusammengedrückt bzw. zusammengerollt wird, seitlich in den Innenraum der Biegefederanordnung 15 eingeführt wird und anschließend entspannt wird. Dadurch, dass er auch in der Grundstellung des chirurgischen Clips 10A nicht vollständig entspannt ist, wird er an der Innenfläche der Biegefederanordnung 15 festgeklemmt.

Der chirurgische Clip 10A gemäß diesem Ausführungsbeispiel wird geöffnet, indem die beiden Betätigungsabschnitte 12A, 12B aufeinander zu bewegt werden. Da die beiden Clipbranchen verschränkt sind, entfernen sich dadurch die beiden Klemmabschnitte 11A, 11B. Der Innendurchmesser der Beigefederanordnung 15 verringert sich dadurch, was dazu führt, dass der C-förmige elastische Körper 20 weiter gebogen wird, was eine zusätzliche Last auf die Biegefederanordnung 15 erzeugt und die gesamte Klemmleistung des chirurgischen Clips 10A erhöht. Der elastische C-förmige Körper 20 wirkt wie eine gebogene Blattfeder, die parallel zu der Biegefederanordnung 15 geschaltet ist und diese unterstützt. Die Innenflächen der Klemmabschnitte 11A, 11B sind bei diesem Ausführungsbeispiel atraumatisch ausgebildet.

Der Spalt zwischen den beiden Enden 21, 22 des C-förmigen elastischen Körpers 20 ist so gewählt, dass die beiden Enden 21, 22 nicht während des Öffnungsvorgangs aneinander stoßen, was zu einer Begrenzung des Öffnungswinkels des Clips 10A führen würde. Bei dem vorliegenden Ausführungsbeispiel ist der Öffnungswinkel durch die Geometrie des Schlitzabschnitts 17 und des Abschnitts mit verringerter Breite 18 begrenzt. Fehlt eine solche Öffnungswinkelbegrenzung und steht zu Befürchten, dass die Biegefederanordnung 15 aufgrund einer zu weiten Öffnung des Mauls plastizieren könnte, was dazu führt, dass das Maul nicht mehr ganz oder zumindest nicht mehr mit der vollen Last schließt, so kann der C-förmige elastische Körper 20 mit seinen beiden Enden 21, 22 als Öffnungswinkelbegrenzung bemessen werden.

Ein zweites Ausführungsbeispiel der vorliegenden Erfindung ist unter Bezugnahme auf die Fig. 1C und 1D im Detail beschrieben. Da der chirurgische Clip 10B ähnlich aufgebaut ist wie der chirurgische Clip 10A gemäß dem ersten Ausführungsbeispiel wird hier vorwiegend auf die Unterschiede zum ersten Ausführungsbeispiel eingegangen, die nicht diskutierten Merkmale sind identisch wie bei dem ersten Ausführungsbeispiel.

An Stelle eines einfachen C-förmigen elastischen Körpers 20 ist bei diesem Ausführungsbeispiel ein C-förmiger elastischer Körper 30 mit je einer Halteeinrichtung 32 und 33 an dessen Stirnseiten vorgesehen. Bei diesem Ausführungsbeispiel erstrecken sich die beiden Halteeinrichtungen 32, 33 jeweils entlang der gesamten Umfangsfläche 31 des C-förmigen elastischen Körpers 30. Die beiden Halteeinrichtungen 32, 33 bilden somit je eine Art Bund, der so weit von dem elastischen C-förmigen Körper 30 nach außen vorsteht, dass der Biegefederabschnitt 15 des chirurgischen Clips 10B vollständig zwischen den beiden Bünden 23, 33 aufgenommen ist.

Der Bund 32 ist dabei einstückig mit dem C-förmigen elastischen Körper 31 ausgebildet, indem ein Bund aus Kunststoff an einen metallischen C-förmigen Körper 31 angespritzt wird. Dieses Bauteil wird dann in die Biegefederanordnung 15 eingesetzt und anschließend wird der zweite Bund 32 an dem C-förmigen Körper 31 angebracht. Im vorliegenden Fall wird der Bund 32, der ebenfalls aus Kunststoff ausgebildet ist, auf den C-förmigen Körper 31 aufgeschoben. Dabei hat der Innendurchmesser des Bunds bzw. der Halteeinrichtung 32 ein gewisses Untermaß, um die Haftung an dem Körper 31 sicher zu stellen.

Wenn für die Halteeinrichtungen 32, 33 und für den elastischen Körper 31 dasselbe Material verwendet wird, können die Bauteile auch gut verschweißt werden. Insbesondere lässt sich das bei metallischen Bauteilen oder Kunststoffbauteilen gut bewerkstelligen.

Die Abmessungen und das Material der Halteeinrichtungen 32, 33 hat bei einer Ausbildung entsprechend dem vorliegenden Ausführungsbeispiel einen großen Einfluss auf die Elastizität bzw. das Biegeverhalten des jeweiligen elastischen Körpers 30, 40, 50. Um den Einfluss der Halteeinrichtungen 32, 33 auf das Biegeverhalten des elastischen Körpers 30, 40, 50 zu verringern, können die Halteeinrichtungen 32, 33 von außen geschlitzt werden.

Da bei diesem Ausführungsbeispiel zwei Halteeinrichtungen vorgesehen sind, welche den elastischen Körper 30 in der Biegefederanordnung 15 sichern, kann der elastische Körper 30 bzw. der C-förmige Körper 31 so dimensioniert sein, dass er in der Grundstellung des chirurgischen Clips 10B, wie sie in Fig. 1B gezeigt ist, vollkommen entspannt ist. Der elastische Körper 30 kann also so dimensioniert werden, dass er entweder schon in der Grundstellung des Clips 10B eine Last auf die Biegefederanordnung 15 aufbringt, er eine Last sofort auf die Biegefederanordnung 15 aufbringt, wenn die Klemmabschnitte 11A, 11B aus der Grundstellung heraus bewegt werden, oder eine Last erst nach einem gewissen Weg der Klemmabschnitte 11A, 11B aus der Grundstellung heraus auf die Biegefederanordnung 15 aufbringt. Auf diese Weise kann der Weg-Kraft-Verlauf der Maulöffnung des Clips 10B eingestellt werden.

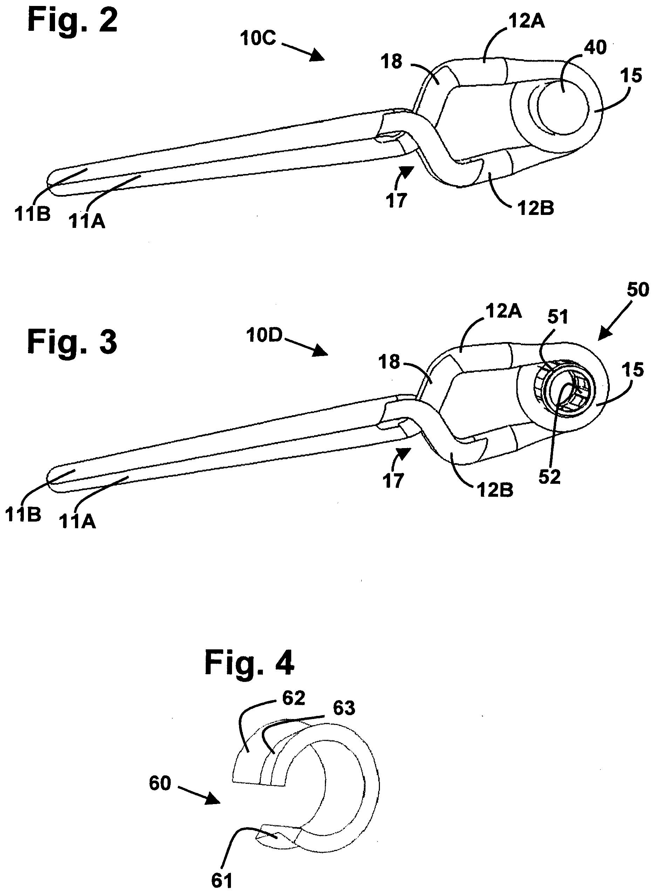

Ein drittes Ausführungsbeispiel der vorliegenden Erfindung ist im Folgenden unter Bezugnahme auf die Fig. 2 im Detail beschrieben. Bei diesem Ausführungsbeispiel ist ein elastischer Körper 40 in der Biegefederanordnung 15 vorgesehen. Der elastische Körper dieses Ausführungsbeispiels besteht aus einem Vollkörper aus Gummi, es kann aber auch ein Vollkörper aus einem Verbundmaterial sein, beispielsweise einem Schichtaufbau aus Gummi und Metall. Dieser Verbundwerkstoff kann entweder aus ebenen Schichten aufgebaut sein oder aus einer Mehrzahl von ineinander geschobenen metallenen Rohren bestehen, die mit Gummi vergossen sind. Prinzipiell kann jede Form von Verbundmaterial eingesetzt werden, aber ein annähernd isotroper Werkstoff ist einem orthotropen Werkstoff im Allgemeinen vorzuziehen, da bei einem isotropen Werkstoff nicht auf dessen Lage in der Biegefederanordnung 15 geachtet werden muss.

Bei diesem Ausführungsbeispiel befindet sich der elastische stabförmige Körper 40 entlang seines gesamten Umfangs in Kontakt mit der Innenfläche der Biegefederanordnung 15. Zudem ist entlang des Umfangs des elastischen Körpers 40 eine Nut (in Fig. 2 nicht gezeigt) ausgebildet, welche sicher stellt, dass der elastische Körper 40 auch in der Grundstellung des Clips 10C nicht aus der Biegefederanordnung 15 heraus fällt.

Selbstverständlich kann auch dieser elastische Körper 40 mit einer oder zwei Halteeinrichtungen versehen sein. Diese können hier beispielsweise aus zwei kreisförmigen Platten bestehen, welche an den Stirnseiten des Körpers 40 angebracht oder befestigt sind.

Ein viertes Ausführungsbeispiel der vorliegenden Erfindung ist im Folgenden unter Bezugnahme auf die Fig. 3 im Detail beschrieben. Bei dem vierten Ausführungsbeispiel ist gegenüber dem dritten Ausführungsbeispiel ein Federkäfig 50 statt eines stabförmigen elastischen Körpers 40 vorgesehen.

Der Federkäfig besteht aus axialen Stäben 52, die beiderseits der Biegefederanordnung 15 an einem Trägerbauteil 51 befestigt sind. Die elastische Wirkung des Federkäfigs 50 wird hauptsächlich dadurch erzielt, dass sich die axialen Stäbe (axial bezieht sich hier auf die Achsrichtung des Federkäfigs) zur Achse des Federkäfigs hin verbiegen können. Wenn ein steiferer Federkäfig 50 erforderlich ist, können mehr axiale Stäbe 52 vorgesehen werden, die Stäbe 52 selbst können steifer ausgebildet werden oder es können zusätzliche ringförmige Bauteile vorgesehen sein, welche die einzelnen Stäbe 52 im Bereich der Biegefederanordnung 15 miteinander verbinden. Der Federkäfig 50 kann aber zum Beispiel auch mit einem Kunststoffmaterial wie beispielswese einem Gummi entsprechend dem dritten Ausführungsbeispiel vergossen werden, um das Elastizitätsmodul zu erhöhen. Wenn die Trägerbauteile 51 einen größeren Durchmesser als den Innendurchmesser der Biegefederanordnung 15 aufweisen, dienen sie zugleich als Halteeinrichtungen.

Ein elastischer C-förmiger Ring entsprechend einem fünften Ausführungsbeispiel ist im Folgenden unter Bezugnahme auf die Fig. 4 im Detail beschrieben.

Der C-förmige elastische Körper 60 gemäß diesem Ausführungsbeispiel weist eine umlaufende V-förmige Nut 61 auf. Wird der Körper in einer Biegefederanordnung angeordnet, so sorgt die Vorspannung des C-förmigen Körpers in Zusammenarbeit mit den geneigten bzw. schrägen Flächen 62, 63 dafür, dass sich der elastische Körper 60 relativ zu der Biegefederanordnung 15 in axialer Richtung zentriert. Auf diese Weise kann leicht sicher gestellt werden, dass der C-förmige Körper 60 korrekt positioniert ist.

Viele Merkmale, die hier zu den einzelnen Ausführungsbeispielen beschrieben sind, lassen sich aber auch in Kombination mit anderen Ausführungsbeispielen verwirklichen, so können z.B. verschiedenste Halteeinrichtungen 32, 33 mit verschiedensten elastischen Körpern 20, 30, 40, 50, 60 kombiniert werden. Ebenso sind die angegebenen Materialien für alle Ausführungsbeispiele anwendbar. Insgesamt sind alle Merkmale der einzelnen Ausführungsbeispiele beliebig geeignet kombinierbar.

## Patentansprüche

1. Chirurgischer Clip mit zwei Clipbranchen jeweils aufweisend einen Klemmabschnitt und einen Betätigungsabschnitt sowie mit einer Biegefederanordnung, über welche die beiden Clipbranchen einstückig miteinander gekoppelt sind, wobei die Biegefederanordnung mehr als eine halbe Wicklung aufweist
**dadurch gekennzeichnet, dass**
ein elastischer Körper so in der Biegefederanordnung angeordnet ist, dass er durch einen Öffnungsvorgang des chirurgischen Clips verformbar ist, und zwischen dem elastischen Körper und der Biegefederanordnung drei oder mehr Kontaktstellen ausgebildet sind, wobei die drei oder mehr Kontaktstellen über einen Winkelbereich von mindestens 180° verteilt sind.

2. Chirurgischer Clip nach Anspruch 1,
**dadurch gekennzeichnet, dass**
der elastische Körper ein im Wesentlichen C-förmiger elastischer Körper ist, der so in der Biegefederanordnung angeordnet ist, dass er sich im Wesentlichen entlang seiner gesamten Außenfläche in Kontakt mit der Innenfläche der Biegefederanordnung befindet.

3. Chirurgischer Clip nach Anspruch 2,
**dadurch gekennzeichnet, dass**
der im Wesentliche C-förmige elastische Körper zu den Klemmabschnitten der Clipbranchen hin offen ist.

4. Chirurgischer Clip nach Anspruch 2 oder 3,
**dadurch gekennzeichnet, dass**
der im Wesentliche C-förmige elastische Körper eine umlaufende Nut an seiner Außenumfangsfläche aufweist, die daran angepasst ist, die Biegefederanordnung zumindest teilweise aufzunehmen.

5. Chirurgischer Clip nach Anspruch 1,
**dadurch gekennzeichnet, dass**
der elastische Körper ein im Wesentlichen stabförmiger elastischer Körper ist, der so in der Biegefederanordnung angeordnet ist, dass die Kontaktstellen zwischen der Mantelfläche des elastischen stabförmigen Körpers und der Innenfläche der Biegefederanordnung entlang des gesamten Umfangs des elastischen stabförmigen Körpers vorzugsweise gleichmäßig verteilt sind.

6. Chirurgischer Clip nach Anspruch 5,
**dadurch gekennzeichnet, dass**
der im Wesentlichen stabförmige elastische Körper einen kreisförmigen, einen vieleckigen oder einen sternförmigen Querschnitt aufweist.

7. Chirurgischer Clip nach einem der vorangehenden Ansprüche,
**dadurch gekennzeichnet, dass**
der elastische Körper ein Federkäfig ist, der so in der Biegefederanordnung angeordnet ist, dass er sich im Wesentlichen entlang seines gesamten Umfangs mit seiner Außenfläche in Kontakt mit der Innenfläche der Biegefederanordnung befindet.

8. Chirurgischer Clip nach einem der vorangehenden Ansprüche,
**dadurch gekennzeichnet, dass**
an wenigstens einer Stirnfläche des elastischen Körpers eine Halteeinrichtung vorgesehen ist, welches über den Innenradius der Biegefederanordnung nach außen vorsteht.

9. Chirurgischer Clip nach Anspruch 8,
**dadurch gekennzeichnet, dass**
sich die Halteeinrichtung im Wesentlichen entlang des gesamten Außenumfangs des elastischen Körpers erstreckt.

10. Chirurgischer Clip nach einem der vorangehenden Ansprüche,
**dadurch gekennzeichnet, dass**
der elastische Körper einen in axialer Richtung veränderlichen Querschnitt derart aufweist, dass er beiderseits der Biegefederanordnung einen gegenüber dem zentralen Bereich, an dem er sich in Kontakt mit der Innenfläche der Biegefederanordnung befindet, vergrößerten Umkreis aufweist.

11. Chirurgischer Clip nach einem der vorangehenden Ansprüche,
**dadurch gekennzeichnet, dass**
die Biegefederanordnung eineinhalb Wicklungen aufweist.

12. Chirurgischer Clip nach einem der vorangehenden Ansprüche,
**dadurch gekennzeichnet, dass**
die beiden Clipbranchen zwischen dem jeweiligen Klemmabschnitt und dem jeweiligen Betätigungsabschnitt verschränkt sind.

13. Chirurgischer Clip nach Anspruch 12,
**dadurch gekennzeichnet, dass**
eine der beiden Clipbranchen im Bereich der Verschränkung geschlitzt ist und sich die andere der beiden Clipbranchen durch diesen Schlitz erstreckt und durch den Schlitz geführt ist.

14. Chirurgischer Clip nach einem der vorangehenden Ansprüche,
**dadurch gekennzeichnet, dass**
der Clip zumindest im Bereich der Biegefederanordnung beschichtet oder lackiert ist, um die Reibung zwischen der Biegefederanordnung und dem elastischen Körper zu verringern.

15. Chirurgischer Clip nach einem der vorangehenden Ansprüche,
**dadurch gekennzeichnet, dass**
der elastische Körper aus Kunststoff oder Metall ausgebildet ist, vorzugsweise aus Federstahl oder einer Titanlegierung.

## Claims

1. Surgical clip with two clip branches each having a clamping portion and an actuating portion and also with a flexible spring arrangement, by way of which the two clip branches are integrally coupled to one another, wherein the flexible spring arrangement has more than a half winding,
**characterized in that**
an elastic body is arranged in the flexible spring arrangement in such a way that it can be deformed by an opening operation of the surgical clip, and three or more contact points are formed between the elastic body and the flexible spring arrangement, wherein the three or more contact points are distributed over an angular range of at least 180°.

2. Surgical clip according to Claim 1,
**characterized in that**
the elastic body is a substantially C-shaped elastic body, which is arranged in the flexible spring arrangement in such a way that it is in contact with the inner face of the flexible spring arrangement substantially along its entire outer face.

3. Surgical clip according to Claim 2,
**characterized in that**
the substantially C-shaped elastic body is open toward the clamping portions of the clip branches.

4. Surgical clip according to Claim 2 or 3,
**characterized in that**
the substantially C-shaped elastic body has a circumferential groove on its outer circumferential face, said groove being adapted to at least partially receive the flexible spring arrangement.

5. Surgical clip according to Claim 1,
**characterized in that**
the elastic body is a substantially bar-shaped elastic body, which is arranged in the flexible spring arrangement in such a way that the contact points between the lateral face of the elastic bar-shaped body and the inner face of the flexible spring arrangement are preferably distributed uniformly along the entire circumference of the elastic bar-shaped body.

6. Surgical clip according to Claim 5,
**characterized in that**
the substantially bar-shaped elastic body has a circular, a polygonal or a star-shaped cross section.

7. Surgical clip according to one of the preceding claims,
**characterized in that**
the elastic body is a spring cage, which is arranged in the flexible spring arrangement in such a way that it is in contact with the inner face of the flexible spring arrangement with its outer face substantially along its entire circumference.

8. Surgical clip according to one of the preceding claims,
**characterized in that**
a holding device is provided on at least one end face of the elastic body and protrudes outward beyond the inner radius of the flexible spring arrangement.

9. Surgical clip according to Claim 8,
**characterized in that**
the holding device extends substantially along the entire outer circumference of the elastic body.

10. Surgical clip according to one of the preceding claims,
**characterized in that**
the elastic body has a variable cross section in the axial direction, in such a manner that, on both sides of the flexible spring arrangement, it has a circumscribed circle which is enlarged compared to the central region, at which it is in contact with the inner face of the flexible spring arrangement.

11. Surgical clip according to one of the preceding claims,
**characterized in that**
the flexible spring arrangement has one and a half windings.

12. Surgical clip according to one of the preceding claims,
**characterized in that**
the two clip branches are crossed between the respective clamping portion and the respective actuating portion.

13. Surgical clip according to Claim 12,
**characterized in that**
one of the two clip branches is slotted in the region of the crossing and the other one of the two clip branches extends through said slot and is guided through the slot.

14. Surgical clip according to one of the preceding claims,
**characterized in that**
the clip is coated or painted at least in the region of the flexible spring arrangement in order to reduce the friction between the flexible spring arrangement and the elastic body.

15. Surgical clip according to one of the preceding claims,
**characterized in that**
the elastic body is formed from plastic or metal, preferably from spring steel or a titanium alloy.

## Revendications

1. Pince chirurgicale avec deux branches de pince présentant respectivement une partie de serrage et une partie d'actionnement ainsi qu'avec un agencement de ressort de flexion, par lequel les deux branches de pince sont couplées l'une à l'autre en une seule pièce, dans laquelle l'agencement de ressort de flexion présente plus qu'une demi-spire,
**caractérisée en ce qu'**un corps élastique est disposé dans l'agencement de ressort de flexion, de telle manière qu'il puisse être déformé par une opération d'ouverture de la pince chirurgicale, et trois points de contact, ou plus, sont formés entre le corps élastique et l'agencement de ressort de flexion, dans laquelle les trois points de contact, ou plus, sont répartis sur une zone angulaire d'au moins 180°.

2. Pince chirurgicale selon la revendication 1,
**caractérisée en ce que** le corps élastique est un corps élastique essentiellement en forme de C, qui est disposé dans l'agencement de ressort de flexion de telle manière qu'il se trouve en contact avec la face intérieure de l'agencement de ressort de flexion essentiellement le long de toute sa face extérieure.

3. Pince chirurgicale selon la revendication 2,
**caractérisée en ce que** le corps élastique essentiellement en forme de C est ouvert en direction des parties de serrage des branches de pince.

4. Pince chirurgicale selon la revendication 2 ou 3,
**caractérisée en ce que** le corps élastique essentiellement en forme de C présente une rainure périphérique sur sa face périphérique extérieure, qui est adaptée pour recevoir au moins en partie l'agencement de ressort de flexion.

5. Pince chirurgicale selon la revendication 1,
**caractérisée en ce que** le corps élastique est un corps élastique essentiellement en forme de barre, qui est disposé dans l'agencement de ressort de flexion, de telle manière que les points de contact entre la face latérale du corps élastique en forme de barre et la face intérieure de l'agencement de ressort de flexion soient répartis de préférence uniformément le long de tout le pourtour du corps élastique en forme de barre.

6. Pince chirurgicale selon la revendication 5,
**caractérisée en ce que** le corps élastique essentiellement en forme de barre présente une section transversale ronde, polygonale ou en forme d'étoile.

7. Pince chirurgicale selon l'une quelconque des revendications précédentes, **caractérisée en ce que** le corps élastique est une cage de ressort, qui est disposée dans l'agencement de ressort de flexion, de telle manière qu'elle se trouve en contact par sa face extérieure avec la face intérieure de l'agencement de ressort de flexion essentiellement le long de toute sa périphérie.

8. Pince chirurgicale selon l'une quelconque des revendications précédentes, **caractérisée en ce qu'**il est prévu sur au moins une face frontale du corps élastique un dispositif de retenue, qui est saillant vers l'extérieur au-delà du rayon intérieur de l'agencement de ressort de flexion.

9. Pince chirurgicale selon la revendication 8,
**caractérisée en ce que** le dispositif de retenue s'étend essentiellement le long de toute la périphérie extérieure du corps élastique.

10. Pince chirurgicale selon l'une quelconque des revendications précédentes, **caractérisée en ce que** le corps élastique présente une section transversale variable en direction axiale, de telle manière qu'il présente de part et d'autre de l'agencement de ressort de flexion un périmètre agrandi par rapport à la région centrale, sur laquelle il se trouve en contact avec la face intérieure de l'agencement de ressort de flexion.

11. Pince chirurgicale selon l'une quelconque des revendications précédentes, **caractérisée en ce que** l'agencement de ressort de flexion présente une spire et demie.

12. Pince chirurgicale selon l'une quelconque des revendications précédentes, **caractérisée en ce que** les deux branches de pince sont croisées entre la partie de serrage respective et la partie d'actionnement respective.

13. Pince chirurgicale selon la revendication 12,
**caractérisée en ce qu'**une des deux branches de pince est fendue dans la région du croisement et l'autre des deux branches de pince s'étend à travers cette fente et est guidée à travers la fente.

14. Pince chirurgicale selon l'une quelconque des revendications précédentes, **caractérisée en ce que** la pince est revêtue ou laquée au moins dans la région de l'agencement de ressort de flexion, afin de réduire le frottement entre l'agencement de ressort de flexion et le corps élastique.

15. Pince chirurgicale selon l'une quelconque des revendications précédentes, **caractérisée en ce que** le corps élastique est fabriqué en matière plastique ou en métal, de préférence en acier à ressort ou en un alliage de titane.
